# EUROPEAN PATENT SPECIFICATION

(11) **EP 2 180 834 B1**
(45) Date of publication and mention of the grant of the patent: **04.05.2011**
(21) Application number: 08803411.1
(22) Date of filing: 29.08.2008
(51) Int. Cl.: A61B 8/08, A61B 8/12

(54) **AUTOMATED MONITORING OF MYOCARDIAL FUNCTION BY ULTRASONIC TRANSDUCERS POSITIONED ON THE HEART**
AUTOMATISIERTE ÜBERWACHUNG DER HERZMUSKELFUNKTION MIT AUF DEM HERZ POSITIONIERTEN ULTRASCHALLWANDLERN
SURVEILLANCE AUTOMATIQUE DE LA FONCTION MYOCARDIQUE A L'AIDE DE TRANSDUCTEURS A ULTRASONS PLACES SUR LE COEUR

(30) Priority: 30.08.2007 DK 200701236
(43) Date of publication of application: 05.05.2010
(73) Proprietor: Oslo Universitetssykehus HF, 0514 Oslo (NO)
(72) Inventor: ELLE, Ole Jakob, N-0478 Oslo (NO); FOSSE, Erik, N-0458 Oslo (NO); IHLEN, Halfdan, N-0272 Oslo (NO); ESPINOZA, Andreas, N-1363 Høvik (NO); HOFF, Lars, N-3154 Tolvsrød (NO)
(74) Representative: Plougmann & Vingtoft A/S
(86) International application number: PCT/EP2008/061421
(87) International publication number: WO 2009/027522

(56) References cited:
- EP-A- 0 503 839
- US-A1- 2003 013 964
- MATRE ET AL: "Multilayer radial systolic strain can identify subendocardial ischemia: An experimental tissue Doppler imaging study of the porcine left ventricular wall" EUROPEAN JOURNAL OF ECHOCARDIOGRAPHY, ELSEVIER, [Online] vol. 8, no. 6, 26 July 2006 (2006-07-26), pages 420-430, XP022312647 ISSN: 1525-2167
- HASHIMOTO I ET AL: "Myocardial strain rate is a superior method for evaluation of left ventricular subendocardial function compared with tissue Doppler imaging" JOURNAL OF THE AMERICAN COLLEGE OF CARDIOLOGY, ELSEVIER, NEW YORK, NY, US, vol. 42, no. 9, 5 November 2003 (2003-11-05), pages 1574-1583, XP004640788 ISSN: 0735-1097
- MATRE ET AL: "Radial Strain Gradient Across the Normal Myocardial Wall in Open-chest Pigs Measured with Doppler Strain Rate Imaging" JOURNAL OF THE AMERICAN SOCIETY OF ECHOCARDIOGRAPHY, MOSBY-YEAR BOOK, INC. ST. LOUIS, MO, US, vol. 18, no. 10, 1 October 2005 (2005-10-01), pages 1066-1073, XP005218029 ISSN: 0894-7317

## Description

### Field of the invention

The present invention relates to estimating cardiac pumping capacity, in particular a myocardial function monitoring system applying data recorded by ultrasonic transducers positioned on the heart.

### Background of the invention

During and after cardiac surgery, it is of interest to monitor the performance of the heart and its responses to various forms of treatment. For example, an important mechanism for haemodynamic instability in the post operative period after aorta-coronary bypass surgery is graft occlusion. The regional myocardial ischaemia induced by this occlusion is often difficult to detect with present bedside monitoring techniques. Also, a sensitive technique for detecting regional ischaemia is strongly warranted during the surgical procedure in off-pump by-pass procedures.

There is a need for new monitoring techniques of regional myocardial function in the postoperative period after cardiac surgery. Conventional monitoring techniques as ECG and blood pressure monitoring are of limited sensitivity and specificity. Established modalities as CT and MR are not suitable for continuous post-operative monitoring. Non-invasive echocardiographic methods with 2-D speckle tracking technique or Tissue Doppler Imaging are sensitive methods for detection of myocardial ischaemia, see e.g. *"*Grading of myocardial dysfunction by tissue Doppler echocardiography" Skulstad et al. J. American College of Cardiology 47: 1672-82, 2006. However, such techniques require a skilled operator and do therefore not allow continuous monitoring of myocardial function.

Some older references describe using transducers fastened on the heart. US 4,947,854 and *"*Doppler measurement of myocardial thickening with a single epicardial transducer" Hartley et al., Am J Physiol. Heart Circ Physiol. 245: 1066-1072, 1983 both describe two ultrasound transducers fastened to the myocardium, using one crystal for Doppler measurements of blood flow in coronary vessels and one crystal for measuring thickening of the chamber wall, the crystals are combined in a single probe.

There exist implantable sonomicrographic crystals for real-time monitoring of regional myocardial function, but for experimental use only (e.g *"*Post-systolic Shortening in Ischemic Myocardium - Active Contraction or Passive Recoil?" Skulstad et. Al, Circulation, 106: 718-724, 2002). Implantation of the crystals is not atraumatic or indifferent to the myocardium.

Also, techniques for estimating ischemia from strain rate determined from TDI measurements have been described in e.g. Matre et al. "Multilayer radial systolic strain can identify subendocardial ischemia: An experimental tissue Doppler imaging study of the porcine left ventricular wall" Eup. J. Echocar. 8(6), 420-430, and Hashimoto et al. (2003) "Myocardial Strain Rate Is a Superiour Method for Evaluation of Left Ventricular Subendocardial Function Compared With Tissue Doppler Imaging" Journ. of the American College of Cardiology, Elsevier, Vol 32, No. 9 (2003), 1574-1583.

Finally, US 5,188,106 describes, in relation to Figures 8A-C, a transducer implanted in the right ventricle for detecting myofibril motion.

It is a disadvantage of the above prior art techniques that no automated quantification of the signals is provided, which is needed if it shall be used in a postoperative care unit, especially where a multiple of patient data are continuously monitored.

### Summary of the invention

The object of the invention is to provide a method and a post-operative care unit for analysing and quantifying an ultrasound tissue Doppler imaging (TDI) signal from a transducer fastened on the myocardium. The quantification may result in a parameter or a graphical representation that indicates regional cardiac ischaemia or correlates with global hypokinetic heart function, thereby making the method and care unit suited for continuous postoperative monitoring.

In a first embodiment, the invention provides a method for automatically analysing an ultrasound TDI signal on a computer and generate a parameter or a graphical representation that indicates regional cardiac ischemia or correlates with global hypokinetic heart function from a first TDI signal obtained by a miniaturized Doppler probe positioned in a relevant coronary artery supply area (e.g. the supply area of the left anterior descending (LAD) coronary artery) on an outer surface part of the myocardium, the method comprising:
- extracting from the first TDI signal a trace corresponding to at least one of tissue velocity, tissue strain, or tissue strain rate;
- correlating the extracted trace with a simultaneously recorded electrocardiogram to:
   o assign sections of the extracted trace to individual cardiac cycles; and to
   o define subsections within a cardiac cycle in the extracted trace corresponding to the early systolic phase and the post-systolic phase;
- reading, in at least the post-systolic phase of the extracted trace, a velocity and/or a strain and/or a strain rate; and
- generating a parameter or a graphical representation which is a function of one of these readings and which indicates regional cardiac ischaemia or correlates with global hypokinetic heart function.

A miniaturized Doppler probe is an ultrasonic transducer with connections, cable outlet, housing etc. that are so small that it may be easily fixed to an inner organ with the cable through the skin.

In a second embodiment, the invention provides a postoperative care unit for automatically analysing an ultrasound TDI signal, the unit comprising means for receiving a first TDI signal obtained by a miniaturized Doppler probe to be positioned in a relevant coronary artery supply area (e.g. the supply area of the LAD coronary artery) on an outer surface part of the myocardium and a simultaneously recorded electrocardiogram; a display; and an electronic processing unit comprising software means configured to:
- extracting from the first TDI signal a trace corresponding to at least one of tissue velocity, tissue strain, or tissue strain rate;
- correlating the extracted trace with the electrocardiogram to
   o assign sections of the extracted trace to individual cardiac cycles; and to
   o define subsections within a cardiac cycle in the extracted trace corresponding to the early systolic phase and the post-systolic phase;
- reading, in at least the post-systolic phase of the extracted trace, a velocity and/or a strain and/or a strain rate;
- generating a parameter or a graphical representation which is a function of one of these readings and which indicates regional cardiac ischaemia or correlates with global hypokinetic heart function; and
- displaying the generated parameter or graphical representation.

In a preferred embodiment, the method and the software means in the care unit also read a velocity and/or a strain and/or a strain rate in the systolic phase of the extracted trace, and generate a parameter or a graphical representation which is a function of both the systolic and post-systolic readings. Including both readings may provide the advantage of improving the correlation leading to a more precise prediction of ischaemia.

A specific implementation of the first and second embodiments is briefly described in the following for the purpose of illustration. In this implementation, miniaturized ultrasonic probes are sutured to the outer surface of the one to three main perfusion areas of the coronary arteries and connected to a computer by leads perforating the skin. Reflected ultrasonic signals from the myocardial wall are recorded as radiofrequency (RF) data and can be presented as so-called M-mode pictures which show how the thickness of the wall varies through the heart cycle.

A stable recording of the inside border line of the wall (the endocardium) is difficult to obtain due to several reasons. The transducer has to keep an approximate 90° angle between the ultrasonic beam and the surface. The angle can vary during the heart contraction, thus adding difference in measured thickness. During contraction the interior lining of the heart will fold, and these foldings may superimpose on the wall inside, thus leading to overestimated wall thickness. Another reason may be that signal quality is poor, so that the border between wall inside and the ventricle lumen is poorly outlined. These problems with detecting the endocardium are the main reasons why automated monitoring of the changes in wall thickness during the heart contractions has not become a clinical tool.

To avoid the endocardial problem wall thickening velocities are calculated from the RF data using the Doppler effect. These Doppler signals are stronger than the ultrasonic signals used for measurement of wall thickness and are therefore more optimal for monitoring myocardial function. Thickening velocities increase gradually when the site of measurement is moved from the wall surface towards the endocardium and velocity measurement must be done in defined depths of the wall. It is also an advantage that myocardial ischaemia reduces peak velocity in all layers through the wall. From a technical point of view the aim is to find a depth of measurement where optimal signals are obtained making this embodiment simple to implement.

Below, a number of preferred embodiments, features or elements for the method of care unit are described. Although described mainly in relation to the method of the first embodiment, these preferred embodiments, features or elements are applicable also to the embodiment of the care unit.

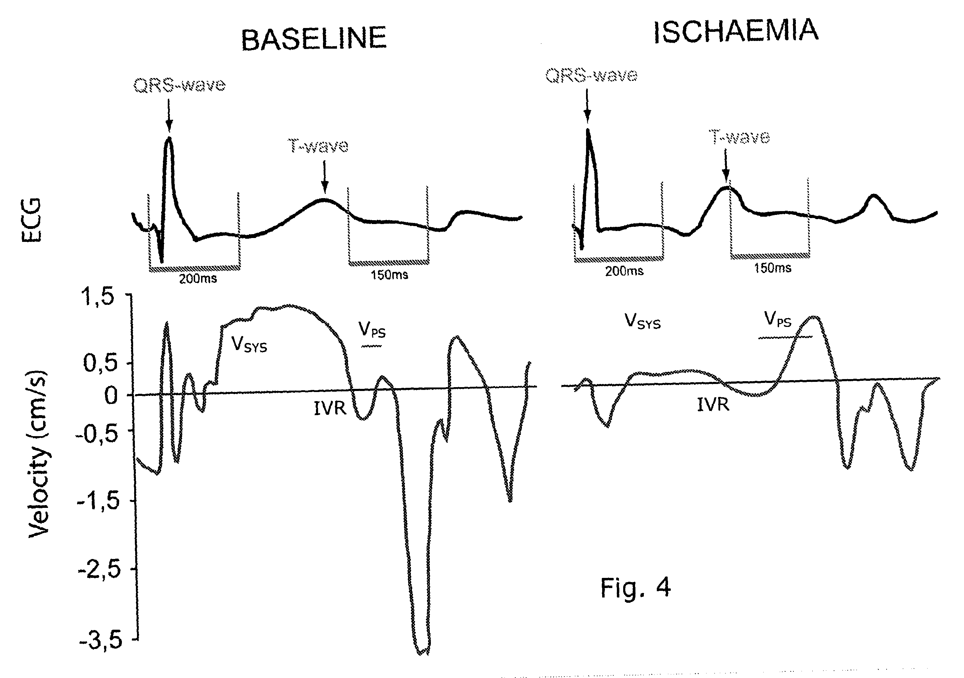

In the present context, the early systolic phase is a period equal to or within the interval from 50ms before the QRS-complex in the ECG (see e.g. Figure 4) signal and until at least 150ms after the peak R, and including the isovolumic contraction phase (IVC). Also, the post-systolic phase is a period equal to or within the interval from aortic valve closure until 150 ms after aortic valve closure, and including the isovolumic relaxation phase (IVR). The timing of aortic valve closure is marked on ECG as the moment the positive systolic velocity declines to zero.

The velocity, strain and/or strain rate value may be read in both the early systolic phase and the post-systolic phase of the extracted trace, so that the reading comprises reading:
- systolic velocity, V_{SYS}, and post-systolic velocity, V_{PS}; and/or
- systolic strain, S_{SYS}, and post-systolic strain, S_{PS}; and/or
- systolic strain rate, SR_{SYS}, and post-systolic strain rate, SR_{PS}.

Thereby, the generated parameter or graphical representation may be a function of both the systolic and the post-systolic reading. In a preferred embodiment, the generated parameter or graphical representation is a function of a difference between the systolic and post-systolic reading, preferably of V_{SYS} - V_{PS}. The value of such function may be displayed as a number or graphically, e.g. as a function of time.

The reading may be a peak value of the trace in the subsection of the extracted trace, or it may be a mean value of the trace in the subsection. Thus the reading may comprise analysing the signal and calculating a derived value.

Instead of a direct generation of the parameter or graphical representation as a mathematical function of the read value(s), it may be preferred to provide empiric signal interpretation data mapping the read velocity, strain or strain rate value, or a derived value or function, to the parameter or graphical representation. Such empiric signal interpretation data may e.g. be a lookup table, an empiric curve or a function which can be used to determine a parameter or graphical representation from a read value. The empiric signal interpretation data is typically determined from previously performed readings during experimentally induced ischaemia or dysfunction, where a degree of seriousness is simultaneously determined by other methods.

In order to verify the correlation with regional cardiac ischaemia or global hypokinetic heart function, signals from further probes fastened elsewhere on the heart may be included in the analysis. Thus, in a preferred embodiment, the analysis is carried out on a second TDI signal obtained by a miniaturized Doppler probe on an outer surface part of the myocardium, and the generated parameter or graphical representation is also a function of one of the readings from the trace extracted from the second TDI signal. Similarly, in yet another embodiment, a TDI signals from a third probe positioned on an outer surface part of the myocardium may be included. The regions of interest will depend on which artery supply areas that have received bypass vessels.

To enable monitoring of the patient over time, it may be preferred to:
- store the parameter or graphical representation generated at a first time; and
- present the stored parameter or graphical representation together with the corresponding parameter or graphical representation generated at later times, thereby enabling direct comparison between parameters or graphical representations at the first time and later times.

An advantage of the present invention is that the generation of the parameter or graphical representation used for monitoring may be automated to a very large degree, such as requiring no human interaction after initial setup. Thus, the embodiments described above may preferably be carried out automatically so that the method and care unit generates the parameter or graphical representation automatically when receiving TDI and ECG signal. In addition, the generation of the parameter or graphical representation may preferably be carried out continuously over a longer period of time. In a preferred embodiment, the parameter or graphical representation is generated from the first TDI signal continuously over a period of at least 24 hours succeeding cardiac surgery.
In order to further automate the monitoring, a threshold value for the generated parameter or graphical representation may preferably be provided, and
o the generated parameter or graphical representation be monitored; and
o an alarm state initiated if the generated parameter or graphical representation passes the threshold value.

For the care unit, incorporation of an alarm in the monitoring may be implemented by further comprising input means allowing an operator to set a threshold value for the generated parameter or graphical representation and means for generating an alarm, wherein electronic processing unit further comprises software means for monitoring the generated parameter or graphical representation and activating the means for generating an alarm if the generated parameter or graphical representation passes the threshold value.

The method and care unit may not only be used for monitoring a state of a patient in order to detect ischaemia or dysfunction due to graft occlusion. In another embodiment, the method and care unit is used to monitor the effect or lack of effect from a medical intervention. Here, the parameter or graphical representation is generated over a period of time including or following intravenous administration of a fluid or a medicament affecting the global hypokinetic heart function. A change in the global hypokinetic heart function during this period of time may be followed and optionally quantified using the parameter or graphical representation. Such a medicament could be infusion of adrenaline to increase the contraction of a hypokinetic heart. This drug may induce ischaemia if given in a too large dose. The ultrasonic measurements of cardiac function would precisely show when the function is normalized and/or if ischaemia occurs and a correct lowest medicament dose can be determined.

In a further embodiment, the invention provides a method for indicating regional cardiac ischaemia or estimating global hypokinetic heart function. This method comprises
- obtaining a first TDI signal from a miniaturized Doppler probe positioned in a relevant coronary artery supply area on an outer surface part of the myocardium;
- extracting from the first TDI signal a trace corresponding to at least one of tissue velocity, tissue strain, or tissue strain rate and reading, in at least a post-systolic phase of the extracted trace, a velocity and/or a strain and/or a strain rate; and
- using the read value, or a derived value or function, to indicate regional cardiac ischaemia or estimate global hypokinetic heart function.

In yet another embodiment, the invention provides the use of a TDI signal for indicating regional cardiac ischaemia or estimating global hypokinetic heart function in accordance with the above method.

The basic idea of the invention is to use TDI signals from one or more miniaturized ultrasonic transducers fastened to the myocardium of a patient to automatically generate parameters or graphical representations that indicate or correlate with myocardial ischaemia or dysfunction. This has the advantage over standard methods using a manually operated ultrasound probe that it can be used continuously over longer periods of time, and thereby be used in post-operation monitoring of patients. In relation to previous disclosures of miniaturized ultrasonic transducers fastened to the heart, the invention provides the advantage of generating parameters or graphical representations that indicate or correlate with myocardial ischaemia or dysfunction.

### Brief description of the figures

Figure 1 is an echo screen showing a M-mode picture displaying wall thickness variations through four cardiac cycles. The broad grey line indicates depth of tissue velocity measurement (See Figure 3B).
Figure 2 is an illustration of a postoperative care unit according to an embodiment of the invention.
Figure 3A echo screen, 3B Doppler screen; In 3B, ECG-curve (upper) and trace of tissue velocity (lower) in indicated depth (broad grey line) in M-mode window (3B). White horizontal and vertical bars inserted to exemplify a relevant time window for measurement of mean systolic and post-systolic velocities, and their approximate values.
Figure 4 shows ECG-synchronized velocity curves in baseline and ischaemic situation.
Figures 5A-B and 6A-B show the changes in (5) systolic and (6) post-systolic tissue velocity from baseline to ischaemia for (A) miniaturized ultrasonic transducer on the myocardium and (B) standard echocardiography using an external manually operated probe.
Figures 7A-B shows the change in a velocity parameter from baseline to ischaemia for (A) miniaturized ultrasonic transducer on the myocardium and (B) standard echocardiography using an external manually operated probe.
Figures 8 and 9 show how changes in wall thickening and (V_{sys}-Vₚₛ) occur when the heart is exposed to global interventions.

### Detailed description of the invention

The invention relates to the analysis and treatment of data from a miniaturized ultrasonic transducer fastened to the myocardium of a patient. Hence, the operative procedure of fastening the transducer is a separate and preceding step which is not part of the invention or covered by the present application/patent.

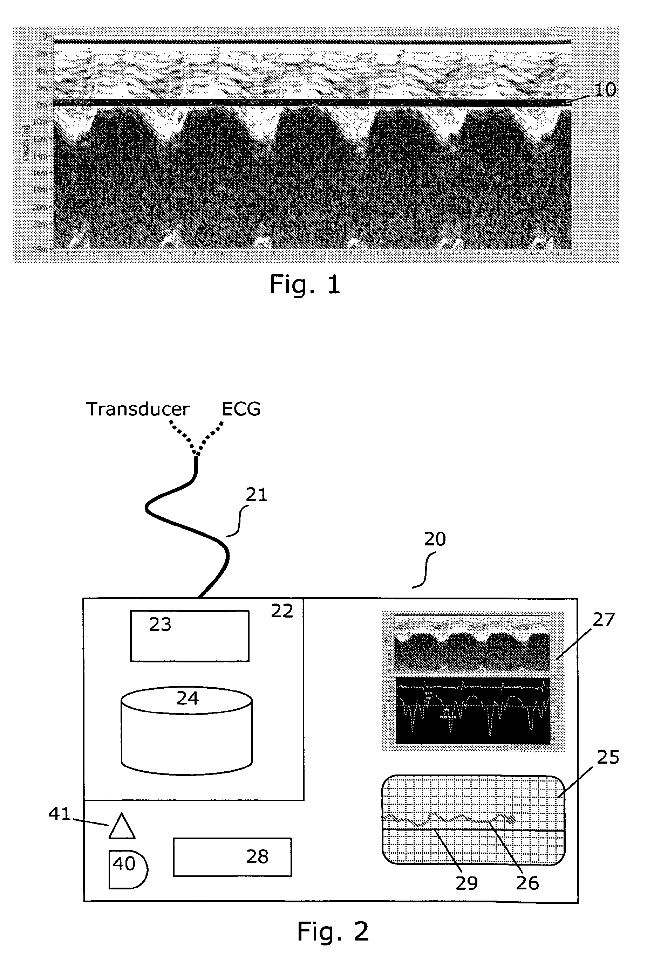

Miniaturized ultrasonic transducers are known from a number of applications, both medical and non-medical, see e.g. US 2006/0116584. When the ultrasonic transducer is sutured to the heart during the operation an optimal position on the wall and depth of measurement is secured by using M-mode echo signals as guidance shown in Figure 1. An optimal position has been obtained when relatively distinct outer and inner surface lines of the wall are seen. Since the miniature ultrasound transducer is a regional monitor of myocardial function, it may be preferable to have two or three miniature ultrasound transducers in order to monitor global function or to confirm the reading from the main transducer. If more than one transducer is to be positioned, it is preferable to have one transducer in each of the one to three by-pass areas of the myocardium.

Figure 2 is a schematic illustration of a postoperative care unit 20 according to an embodiment of the invention, comprising an electronic processing unit 22 such as an integrated computer. The care unit has means 21 for receiving a first TDI signal from the miniaturized ultrasonic transducer, e.g. the one positioned in the supply area of the left anterior descending (LAD) coronary artery on an outer surface part of the myocardium. The means 21 can typically comprise a cable, an input terminal, an analogue to digital converter, and circuitry connecting the means to the electronic processing unit 22 comprising a processor 23 and a memory 24 for storing the received signals. The means 21 also receives a simultaneously recorded electrocardiogram, typically from standard ECG equipment.

The memory 24 of the electronic processing unit 22 holds software means to be executed by the processor 23 for analysing the received signals and generating the parameter or graphical representation as will be described in relation to the method elsewhere.

The care unit 20 further comprises a display 25 for displaying the generated parameter or graphical representation. In the specific example shown in Figure 2, a parameter values is represented graphically as a function of time as curve 26 in display 25.

As an optional feature, the care unit can also have a display 27 for showing the e.g. direct echo and Doppler signals as well as other standard monitoring parameters used in an intensive care unit (electrocardiogram (ECG), blood pressure, heart rate, central venous pressure, O2-tension etc.). These can be used by an operator to ensure that the obtained TDI signals are appropriate, confirming that the transducer is properly fastened and functioning.

In one embodiment, the care unit comprises input means 28 allowing an operator to set a threshold value for the generated parameter or graphical representation, typically a keyboard as shown or a connection 40 to a remote computer. In the specific example shown in Figure 2, the threshold value is shown in the display 25 as line 29, so that the operator can continuously see how close the generated value 26 is to the threshold. The electronic processing unit comprises software means for monitoring the generated value 26 and activating means for generating an alarm if the generated value 26 passes below the line 29. The means for generating an alarm serves to notify an operator or hospital staff on duty. It can be implemented by a loudspeaker and a warning light 41 as shown, or it could be a network connection 40 to a centralized alarm system.

In order to set the threshold value, the operator can define a baseline value, e.g. defined over 10 heart beats. The threshold value can then be set as a fraction or multiple of the baseline, e.g. when an abnormal low peak velocity, a high post-systolic velocity, or a negative value of the function (V_{SYS} - V_{PS}) occurs. The user may select which of these options he wants to use during the monitoring.

In the following, the process of extraction and analysis of a tissue velocity trace from the first TDI signal is described. Although described in relation to tissue velocity, the process applied equally to traces corresponding to tissue strain or tissue strain rate.

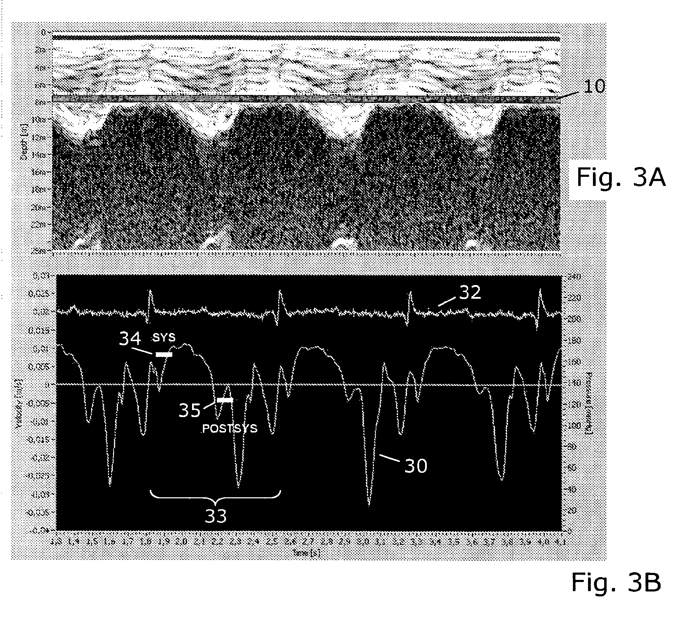

In the intensive care unit 20, the computer transforms the RF signals to velocities at a depth inside the wall, showing a velocity trace, curve 30, on the Doppler screen in Figure 3B. This trace is displayed below the M-mode picture of the wall (Figure 3A). The optimal quality of velocity trace is usually obtained close to the inner surface of the wall. The measuring depth is shown as a grey line 10 in the M-mode picture in Figure 3A. The Doppler screen of Figure 3B also displays a ECG-curve 32.

Using the simultaneously recorded ECG, the extracted trace can be divided into individual sections 33 according to individual cardiac cycles, i.e. heartbeats. Within each section 33, subsections corresponding to the early systolic phase and the post-systolic phase can be defined, shown as time intervals 34 and 35 on the extracted velocity trace 30.

Now, a velocity (and/or strain and/or strain rate) value can be read in the defined intervals, such reading can be performed by an appropriate software application in the velocity trace data. The values can for example be peak or average values within the interval. When such interval 34 is placed at the ejection phase as in Figure 3B, systolic velocity is automatically measured and a peak or mean value, V_{SYS}, can be calculated. The same principle is used for selection of a post-systolic time interval 35 (just after end of systole), thereby acquiring an automated measurement of post-systolic velocity, V_{PS}.

Several different parameters or graphical representations indicating regional cardiac ischaemia or correlating with global hypokinetic heart function exist. In the simplest embodiment, the parameter is the peak or mean value of the post-systolic velocity, V_{PS}. In another embodiment, the parameter is the function V_{SYS} - V_{PS}, i.e. difference between (peak or mean values of) systolic velocity and post-systolic velocity. This value predicts ischaemia with good sensitivity and specificity, and improves the certainty in ischaemia diagnostics as will be shown later.

If more than one transducer, the above procedure is performed for each of the transducers and the display can show three numbers continuously plus a baseline value on the screen. If the alarm is activated due to a low value from one or more of the transducers, this may indicate falling myocardial function. The operator can check the velocity trace to control that the quality of the myocardial velocity signals are adequate. If the quality of the velocity is adequate there are two possible conclusions: When V_{SYS} is abnormal in all three regions of the myocardium with unchanged V_{PS} and only moderate fall in V_{SYS} - V_{PS}, there is strong evidence for a global altered myocardial function due to changes in loading or inotropic conditions. If there are changes in only one of the regions, and most for the V_{SYS} - V_{PS} parameter, there are strong evidences for a regional ischemic event.

### Experiments and clinical studies

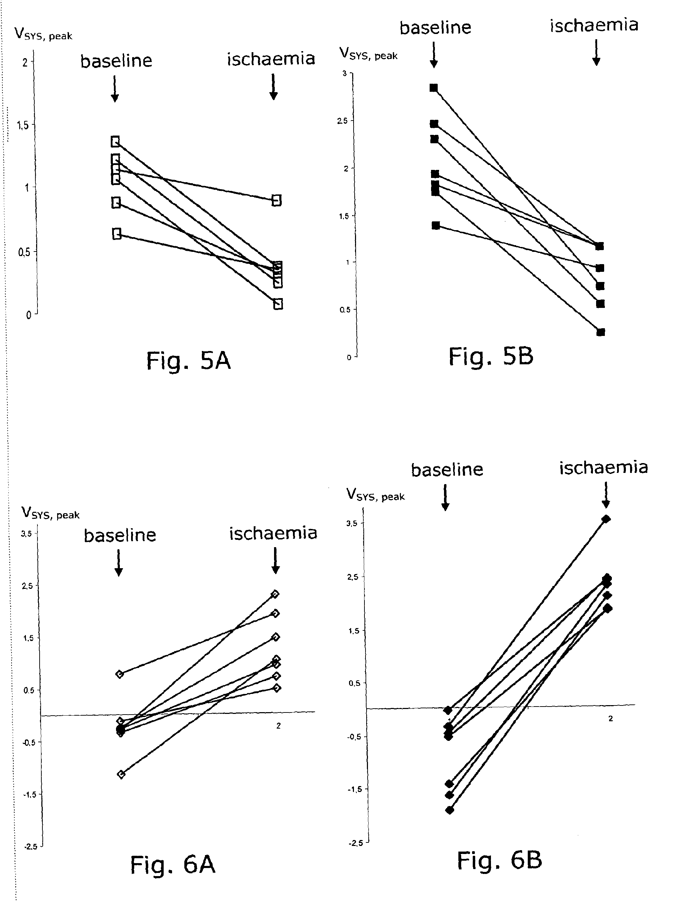

In the following, a number of experiments and clinical studies are described in relation to Figures 4 through 7. These demonstrate the ability of the method and care unit of the invention to detect myocardial ischaemia and/or dysfunction. The results obtained with the miniaturized ultrasonic transducers are shown in Figures 5A-7A. These results are compared to results from standard echocardiographic techniques using a manually operated external ultrasonic probe, shown in corresponding Figures 5B-7B, in order to show the correlation with present techniques. It is, again, noted that the external probes are not suited for continuous postoperative monitoring of myocardial function since they can not be automated or carried out over longer periods of time.

Experiments have been carried out in a porcine model with seven pigs. We used a miniature prototype 5 MHz ultrasonic transducer (GE Vingmed Ultrasound, Norway) sutured to the heart. In later experiments we used a 10 MHz transducer (Imasonic, France). We registered simultaneous ECG, blood pressure (arterial, central venous, left ventricular and left atrial), cardiac output by transit-time flowprobe (Medistim, Norway). 2D echo and tissue Doppler recordings were performed by conventional echocardiography (Vivid 7, GE Vingmed, Norway).

Ultrasonic RF-data were recorded simultaneously and synchronized with ECG and pressure data in a customized sampling programme made in LabView (LabView 8.0, National Instruments, TX).

The miniature transducer was sutured to the surface of the heart in the supply area of the left descending coronary artery (LAD). LAD was occluded by snaring for 60 seconds. Cessation of flow was confirmed by Doppler flow measurement.

For all measurements, baseline measurement were carried out prior to occlusion or intervention to show the sensitivity of the parameter or graphical representation characterizing the state after occlusion or intervention.

Figure 4 show ECG-synchronized tissue velocity curves of the wall thickening velocity at baseline and after 60 seconds of regional ischaemia. The time frames of 200 and 150 ms show the periods where systolic velocity and post-systolic velocity were registered. Systolic velocity (V_{SYS}) was registered as peak value after isovolumic contraction phase (IVC - this being the first spike in the 200 ms window). Post-systolic velocity (V_{PS}) was calculated as a mean of the minimum and maximum deflection on the velocity curve after start of isovolumic relaxation phase (IVR - this starting at negative velocity-shift after systole).

Systolic wall thickening velocity (V_{SYS}) was significantly reduced from baseline to ischaemia after 60 seconds. Simultaneously, post-systolic thickening velocity (V_{PS}) increased (see Figure 4). The reduction in V_{SYS} indicates loss of contractile force in this region due to ischaemia. A delayed contraction probably occurs during the post-systolic period when the left ventricular pressure falls. This may explain explain the striking increase in V_{PS}.

The findings by the fastened miniature probe (Figures 5A, 6A, and 7A) were in accordance with the results by conventional 2-D echocardiography using an external probe (Figures 5B, 6B and 7B). The results are also summarised in the table below showing mean values with standard deviation. P-values indicate a significant change in all values from baseline to ischaemia.

**Table. Peak and postsystolic velocities with V_{sys}-Vₚₛ relationship**

| | | **Baseline (cm/s)** | **Ischemia (cm/s)** | **p** |
|---|---|---|---|---|
| | Systolic velocity (V_{sys}) | 1.0 ± 0.28 | 0.28 ± 0.12 | <0.01 |
| **Miniature probe** | Postsystolic velocity (Vₚₛ) | 0.13 ± 0.56 | 1.57 ± 0.59 | <0.01 |
| | V_{sys}-Vₚₛ | 0.88 ± 0.63 | -1.29 ± 0.57 | <0.01 |
| | Systolic velocity (Vsys) | 1.93 ± 0.47 | 0.81 ± 0.42 | <0.01 |
| **Echocardiography** | Postsystolic velocity (Vₚₛ) | -0.35 ± 0.94 | 2.09 ± 1.07 | <0.01 |
| | V_{sys}-Vₚₛ | 2.28 ±1,10 | -1.67 ± 0.76 | <0.01 |

Figures 5A and B show the parameter V_{SYS, peak}, the peak velocity in the early systolic phase, for baseline and occluded LAD coronary artery. In 5A, a change in the parameter is seen between baseline and ischaemia for all subjects although the parameter values and the relative change varies from subject to subject. There is a good correlation to the external probe measurements in 5B, confirming the validity of the ischaemia indications by the method.

Figure 6A-B shows the changes in post-systolic tissue velocity (including IVR) in the region of ischaemia. In the baseline situation the values for Vₚₛ are negative, except for one subject which is commented later.

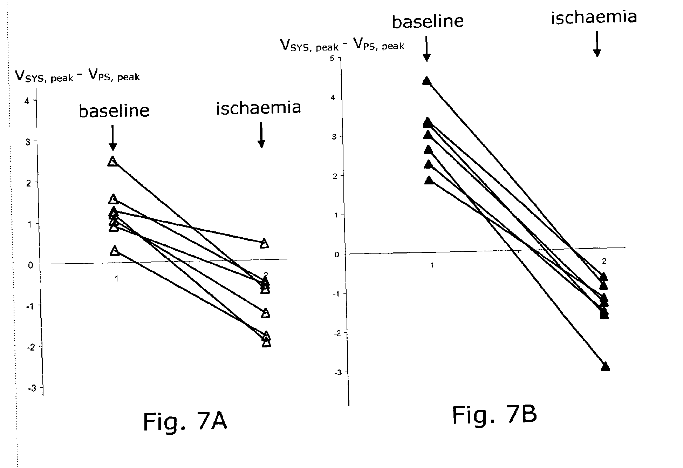

Figures 7A and B show the parameter V_{sys, peak} - V_{PS, peak}, i.e. the difference between the peak velocity in the early systolic phase and in the post-systolic phase, for the embodiments of the invention (7A) and the external probe measurements (7B). Again, the parameters are shown for baseline and occluded LAD coronary artery for the miniaturized probe on the heart and an external probe. In 7A, a clear change in the parameter is seen between baseline and ischaemia for all subjects.

For all subjects but one, the change shifts the sign on the parameter, from positive to negative. Thus, even though the parameter values and the relative change varies from subject to subject, the parameter V_{SYS, peak} - V_{PS, peak}, it is easy to distinct between baseline and ischaemia based solely on the sign of the parameter. In the one subject where the V_{SYS, peak} - V_{PS, peak} does not change to negative in the ischemic situation post-hoc analysis of the transducer placement in a video recording of the experiment reveals that the transducer were situated in a borderline ischemic area. This is interesting, because the trend is very clear also in this subject, even though the transducer is not optimally situated.

Hence, the parameter V_{SYS, peak} - V_{PS}, peak provides a binary indication of ischaemia which does not seem to depend on the absolute parameter values or the subject. Such binary indication is extremely well suited for automation by computers. Again, there is a good correlation to the external probe measurements in 5B and 6B, confirming the validity of the ischaemia indications by the method.

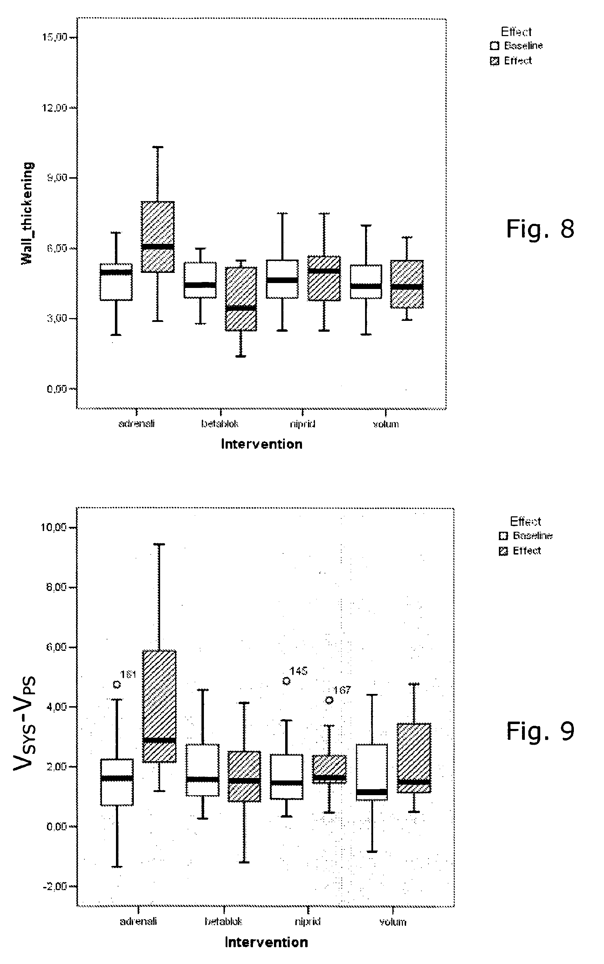

Figures 8 and 9 show how changes in wall thickening and (V_{SYS}-V_{PS}) occur when the heart is exposed to global interventions such as adrenalin, betablockers, niprid and added fluid volume. It demonstrate the potential of using the method or care unit to discriminate between ischaemia and the effect of other interventions.

## Claims

1. A method for automatically analysing an ultrasound tissue Doppler imaging (TDI) signal on a computer (22) and automatically generate a parameter or a graphical representation that indicates regional cardiac ischaemia or correlates with global hypokinetic heart function, the method comprising:
- obtaining a first TDI signal from a miniaturized Doppler probe fastened in a relevant coronary artery supply area on an outer surface part of the myocardium;
- extracting from the first TDI signal a trace corresponding to at least one of tissue velocity, tissue strain, or tissue strain rate;
- correlating the extracted trace with a simultaneously recorded electrocardiogram (30) to
o assign sections of the extracted trace to individual cardiac cycles; and to
o define subsections (34, 35) within a cardiac cycle in the extracted trace corresponding to the early systolic phase and the post-systolic phase;
- reading, in at least the post-systolic phase of the extracted trace, a velocity and/or a strain and/or a strain rate; and
- generating a parameter or a graphical representation which is a function of one of these readings and which indicates regional myocardial ischaemia or correlates with global hypokinetic heart function.

2. The method according to claim 1, wherein the reading comprises reading, in the early systolic phase and the post-systolic phase of the extracted trace:
- systolic velocity, V_{SYS}, and post-systolic velocity, V_{PS}; and/or
- systolic strain, S_{SYS}, and post-systolic strain, S_{PS}; and/or
- systolic strain rate, SR_{SYS}, and post-systolic strain rate, SR_{PS}; and
wherein the generated parameter or graphical representation is a function of the systolic and the post-systolic reading.

3. The method according to claim 2, wherein the generated parameter or graphical representation is a function of a difference between the systolic and post-systolic reading.

4. The method according to any of the preceding claims, wherein the reading comprises reading a peak value of the trace in the subsection of the extracted trace.

5. The method according to any of claims 1-3, wherein the reading comprises reading a mean value of the trace in the subsection of the extracted trace.

6. The method according to any of the preceding claims, further comprising carrying out the steps of the method on a second TDI signal obtained by a miniaturized Doppler probe positioned in a second relevant coronary artery supply area on an outer surface part of the myocardium, and wherein the generated parameter or graphical representation is also a function of one of the readings from the trace extracted from the second TDI signal.

7. The method according to claim 6, further comprising carrying out the steps of the method on a third TDI signal obtained by a miniaturized Doppler probe positioned in a third relevant coronary artery supply area on an outer surface part of the myocardium, and wherein the generated parameter or graphical representation is also a function of one of the readings from the trace extracted from the third TDI signal.

8. The method according to any of the preceding claims, wherein the generation of a parameter or graphical representation which indicates regional cardiac ischaemia or which correlates with global hypokinetic heart function comprises providing empiric signal interpretation data mapping the read velocity, strain or strain rate value, or a derived value or function, to a parameter or graphical representation.

9. The method according to any of the preceding claims, further comprising:
- storing the parameter or graphical representation generated at a first time;
- presenting the stored parameter or graphical representation together with the corresponding parameter or graphical representation generated at later times to enable direct comparison between parameters or graphical representations at the first time and later times.

10. The method according to any of the preceding claims, wherein the parameter or graphical representation is generated from the first TDI signal continuously over a period of at least 24 hours succeeding a surgical intervention.

11. The method according to any of the preceding claims, further comprising providing a threshold value for the generated parameter or graphical representation, and wherein
o the generated parameter or graphical representation is monitored; and
o an alarm state is initiated if the generated parameter or graphical representation passes the threshold value.

12. The method according to any of the preceding claims, wherein the parameter or graphical representation is generated over a period of time including or following intravenous administration of a fluid or a medicament affecting the global hypokinetic heart function, and wherein the method further comprises monitoring a change in the global hypokinetic heart function during this period of time.

13. A postoperative care unit (20) for automatically analysing an ultrasound tissue Doppler imaging (TDI) signal, the unit comprising
means (21) for receiving a first TDI signal obtained by a miniaturized Doppler probe to be positioned in a relevant coronary artery supply area on an outer surface part of the myocardium and a simultaneously recorded electrocardiogram;
a display (27); and
an electronic processing unit (20) comprising software means configured for:
- extracting from the first TDI signal a trace corresponding to at least one of tissue velocity, tissue strain, or tissue strain rate;
- correlating the extracted trace with the electrocardiogram to
o assign sections of the extracted trace to individual cardiac cycles; and to
o define subsections within a cardiac cycle in the extracted trace corresponding to the early systolic phase and the post-systolic phase;
- reading, in at least the post-systolic phase of the extracted trace, a velocity and/or a strain and/or a strain rate;
- generating a parameter or a graphical representation which is a function of one of these readings and which indicates regional cardiac ischaemia or correlates with global hypokinetic heart function; and
- displaying the generated parameter or graphical representation.

14. The postoperative care unit according to claim 13, further comprising input means (28, 40) allowing an operator to set a threshold value for the generated parameter or graphical representation and means (40, 41) for generating an alarm; and wherein electronic processing unit further comprises software means for monitoring the generated parameter or graphical representation and activating the means for generating an alarm if the generated parameter or graphical representation passes the threshold value.

## Patentansprüche

1. Verfahren zur automatischen Analyse eines Gewebedoppler- (TDI)
- Ultraschallsignals an einem Computer (22) und zur automatischen Erzeugung eines Parameters oder einer grafischen Darstellung, der bzw. die eine regionale kardiale Ischämie anzeigt oder mit der globalen hypokinetische Herzfunktion korreliert, wobei das Verfahren umfasst:
- Erhalten eines ersten TDI-Signals von einer Doppler-Miniatursonde, die in einem geeigneten Versorgungsgebiet der Koronararterien an einem Teil der Außenfläche des Myokards befestigt ist;
- Extrahieren einer Kurve aus dem ersten TDI-Signal, die mindestens einer von Gewebegeschwindigkeit, Gewebeverformung oder Gewebeverformungsrate entspricht;
- Korrelieren der extrahierten Kurve mit einem gleichzeitig aufgezeichneten Elektrokardiogramm (30) zur
o Zuordnung von Abschnitten der extrahierten Kurve zu einzelnen kardialen Zyklen und zur
o Festlegung von Unterabschnitten (34, 35) innerhalb eines kardialen Zyklus der extrahierten Kurve, die der frühen systolischen Phase und der postsystolischen Phase entsprechen;
- Ablesen einer Geschwindigkeit und/oder einer Verformung und/oder einer Verformungsrate in mindestens der postsystolischen Phase der extrahierten Kurve und
- Erzeugen eines Parameters oder einer grafischen Darstellung, der bzw. die eine Funktion einer dieser Ablesungen darstellt und der bzw. die eine regionale Myokardischämie anzeigt oder mit der globalen hypokinetische Herzfunktion korreliert.

2. Verfahren nach Anspruch 1, wobei das Ablesen das Ablesen in der frühen systolischen Phase und der postsystolischen Phase der extrahierten Kurve:
- der systolischen Geschwindigkeit V_{SYS} und der postsystolischen Geschwindigkeit V_{PS} und/oder
- der systolischen Verformung S_{SYS} und der postsystolischen Verformung S_{PS} und/oder
- der systolischen Verformungsrate SR_{SYS} und der postsystolischen Verformungsrate SR_{PS} umfasst und
wobei der erzeugte Parameter oder die erzeugte grafische Darstellung eine Funktion der systolischen und der postsystolischen Ablesung ist.

3. Verfahren nach Anspruch 2, wobei der erzeugte Parameter oder die erzeugte grafische Darstellung eine Funktion eines Unterschieds zwischen der systolischen und der postsystolischen Ablesung ist.

4. Verfahren nach einem der vorhergehenden Ansprüche, wobei das Ablesen das Ablesen eines Spitzenwerts der Kurve in dem Unterabschnitt der extrahierten Kurve umfasst.

5. Verfahren nach einem der Ansprüche 1-3, wobei das Ablesen das Ablesen eines Mittelwerts der Kurve in dem Unterabschnitt der extrahierten Kurve umfasst.

6. Verfahren nach einem der vorhergehenden Ansprüche, weiterhin umfassend das Ausführen der Schritte des Verfahrens mit einem zweiten TDI-Signal, das mit einer Doppler-Miniatursonde erhalten wird, die in einem zweiten geeigneten Versorgungsgebiet der Koronararterien an einem Teil der Außenfläche des Myokards befestigt ist, und wobei der erzeugte Parameter oder die erzeugte grafische Darstellung ebenfalls eine Funktion einer der Ablesung von der Kurve ist, die aus dem zweiten TDI-Signal extrahiert wird.

7. Verfahren nach Anspruch 6, weiterhin umfassend das Ausführen der Schritte des Verfahrens mit einem dritten TDI-Signal, das mit einer Doppler-Miniatursonde erhalten wird, die in einem dritten geeigneten Versorgungsgebiet der Koronararterien an einem Teil der Außenfläche des Myokards befestigt ist, und wobei der erzeugte Parameter oder die erzeugte grafische Darstellung ebenfalls eine Funktion einer der Ablesung von der Kurve ist, die aus dem dritten TDI-Signal extrahiert wird.

8. Verfahren nach einem der vorhergehenden Ansprüche, wobei das Erzeugen eines Parameters oder einer grafischen Darstellung, der bzw. die eine regionale kardiale Ischämie anzeigt oder mit der globalen hypokinetische Herzfunktion korreliert, das Bereitstellen von empirischen Daten zur Signalinterpretation umfasst, mit denen der abgelesene Wert für Geschwindigkeit, Verformung oder Verformungsrate oder ein abgeleiteter Wert oder eine abgeleitete Funktion auf einen Parameter oder eine grafische Darstellung abgebildet werden.

9. Verfahren nach einem der vorhergehenden Ansprüche, weiterhin umfassend:
- Speichern des erstmals erzeugten Parameters oder der erstmals erzeugten grafischen Darstellung;
- Präsentieren des gespeicherten Parameters oder der gespeicherten grafischen Darstellung zusammen mit dem entsprechenden Parameter oder der entsprechenden grafischen Darstellung, der bzw. die später erzeugt wurde, um einen direkten Vergleich des ersten und späteren Parameters oder der ersten und späteren grafischen Darstellung zu ermöglichen.

10. Verfahren nach einem der vorhergehenden Ansprüche, wobei der Parameter oder die grafische Darstellung mit dem ersten TDI-Signal kontinuierlich über einen Zeitraum von mindestens 24 Stunden im Anschluss an einen chirurgischen Eingriff erzeugt wird.

11. Verfahren nach einem der vorhergehenden Ansprüche, weiterhin umfassend das Bereitstellen eines Schwellenwerts für den erzeugten Parameter oder die erzeugte grafische Darstellung und wobei
o der erzeugte Parameter oder die erzeugte grafische Darstellung überwacht wird und
o ein Alarmzustand ausgelöst wird, wenn der erzeugte Parameter oder die erzeugte grafische Darstellung den Schwellenwert überschreitet.

12. Verfahren nach einem der vorhergehenden Ansprüche, wobei der Parameter oder die grafische Darstellung über einen Zeitraum erzeugt wird, der die intravenöse Verabreichung einer Flüssigkeit oder eines Arzneimittels, die bzw. das die globale hypokinetische Herzfunktion beeinflusst, umfasst oder sich daran anschließt und wobei das Verfahren weiterhin das Überwachen einer Veränderung der globalen hypokinetischen Herzfunktion während dieses Zeitraums umfasst.

13. Postoperative Pflegeeinheit (20) für die automatische Analyse eines Gewebedoppler- (TDI) -Ultraschallsignals, wobei die Einheit umfasst
Mittel (21) zum Empfang eines ersten TDI-Signals, das von einer Doppler-Miniatursonde erhalten wird, die in einem geeigneten Versorgungsgebiet der Koronararterien an einem Teil der Außenfläche des Myokards befestigt ist, und eines gleichzeitig aufgenommenen Elektrokardiogramms;
eine Anzeige (27) und
eine elektronische Verarbeitungseinheit (20), umfassend Softwaremittel, die konfiguriert sind für das:
- Extrahieren einer Kurve aus dem ersten TDI-Signal, die mindestens einer von Gewebegeschwindigkeit, Gewebeverformung oder Gewebeverformungsrate entspricht;
- Korrelieren der extrahierten Kurve mit dem Elektrokardiogramm zur
o Zuordnung von Abschnitten der extrahierten Kurve zu einzelnen kardialen Zyklen und zur
o Festlegung von Unterabschnitten innerhalb eines kardialen Zyklus der extrahierten Kurve, die der frühen systolischen Phase und der postsystolischen Phase entsprechen;
- Ablesen einer Geschwindigkeit und/oder einer Verformung und/oder einer Verformungsrate in mindestens der postsystolischen Phase der extrahierten Kurve;
- Erzeugen eines Parameters oder einer grafischen Darstellung, die eine Funktion einer dieser Ablesung darstellt und die eine regionale kardiale Ischämie anzeigt oder mit der globalen hypokinetische Herzfunktion korreliert, und
- Anzeige des erzeugten Parameters oder der erzeugten grafischen Darstellung.

14. Postoperative Pflegeeinheit nach Anspruch 13, weiterhin umfassend Eingabemittel (28, 40), mit denen der Bediener einen Schwellenwert für den erzeugten Parameter oder die erzeugte grafische Darstellung eingeben kann, und Mittel (40, 41) zum Erzeugen eines Alarms und wobei die elektronische Verarbeitungseinheit weiterhin Softwaremittel zum Überwachen des erzeugten Parameters oder der erzeugten grafischen Darstellung und zum Aktivieren der Mittel zum Erzeugen eines Alarms umfasst, wenn der erzeugte Parameter oder die erzeugte grafische Darstellung den Schwellenwert überschreitet.

## Revendications

1. Méthode permettant d'analyser automatiquement un signal ultrasonore d'imagerie Doppler tissulaire (IDT) sur un ordinateur (22) et de générer automatiquement une représentation paramétrique ou graphique qui indique une ischémie cardiaque régionale ou est corrélée avec une fonction cardiaque hypokinétique globale, la méthode comprenant les étapes suivantes:
- obtention d'un premier signal d'IDT à l'aide d'une sonde Doppler miniaturisée fixée dans une zone d'irrigation artérielle coronaire appropriée sur une partie de la surface externe du myocarde;
- extraction à partir du premier signal d'IDT d'un tracé correspondant à au moins l'un des éléments suivants: vitesse tissulaire, tension tissulaire ou taux de tension tissulaire;
- corrélation du tracé extrait avec un électrocardiogramme enregistré simultanément (30) pour
o affecter des parties du tracé extrait à des cycles cardiaques individuels ; et pour
o définir des sous-sections (34, 35) d'un cycle cardiaque dans le tracé extrait correspondant au début de la phase systolique et à la phase post-systolique ;
- lecture, dans au moins la phase post-systolique du tracé extrait, d'une vitesse et/ou d'une tension et/ou d'un taux de tension ; et
- production d'une représentation paramétrique ou graphique qui est fonction de l'une de ces lectures et qui indique une ischémie myocardique régionale ou est corrélée avec une fonction cardiaque hypokinétique globale.

2. La méthode selon la revendication 1, dans laquelle la lecture comprend la lecture, au début de la phase systolique et dans la phase post-systolique du tracé extrait, de :
- la vitesse systolique, V_{SYS}, et la vitesse post-systolique, V_{PS} ; et/ou
- la tension systolique, S_{SYS}, et la tension postsystolique, S_{PS} ; et/ou
- le taux de tension systolique, SR_{SYS}, et le taux de tension post-systolique, SR_{PS} ; et
où la représentation paramétrique ou graphique générée est fonction de la lecture systolique et post-systolique.

3. La méthode selon la revendication 2, où la représentation paramétrique ou graphique générée est fonction d'une différence entre la lecture systolique et post-systolique.

4. La méthode selon l'une quelconque des revendications précédentes, où la lecture comprend la lecture d'une valeur maximale du tracé dans la sous-section du tracé extrait.

5. La méthode selon l'une quelconque des revendications 1 à 3, où la lecture comprend la lecture d'une valeur moyenne du tracé dans la sous-section du tracé extrait.

6. La méthode selon l'une quelconque des revendications précédentes, comprenant également la mise en oeuvre des étapes de la méthode sur un deuxième signal d'IDT obtenu à l'aide d'une sonde Doppler miniaturisée positionnée dans une deuxième zone d'irrigation artérielle coronaire appropriée sur une partie de la surface externe du myocarde, et où la représentation paramétrique ou graphique générée est aussi fonction de l'une des lectures provenant du tracé extrait du deuxième signal d'IDT.

7. La méthode selon la revendication 6, comprenant également la mise en oeuvre des étapes de la méthode sur un troisième signal d'IDT obtenu à l'aide d'une sonde Doppler miniaturisée positionnée dans une troisième zone d'irrigation artérielle coronaire appropriée sur une partie de la surface externe du myocarde, et où la représentation paramétrique ou graphique générée est aussi fonction de l'une des lectures provenant du tracé extrait du troisième signal d'IDT.

8. La méthode selon l'une quelconque des revendications précédentes, où la production d'une représentation paramétrique ou graphique qui indique une ischémie cardiaque régionale ou est corrélée avec une fonction cardiaque hypokinétique globale comprend l'apport de données d'interprétation des signaux empiriques établissant une correspondance entre la valeur de vitesse, de tension ou de taux de tension lue, ou une valeur ou fonction dérivée, et une représentation paramétrique ou graphique.

9. La méthode selon l'une quelconque des revendications précédentes, comprenant en outre les étapes suivantes :
- stockage de la représentation paramétrique ou graphique générée dans un premier temps ;
- présentation de la représentation paramétrique ou graphique stockée avec la représentation paramétrique ou graphique correspondante générée ultérieurement pour permettre une comparaison directe entre les représentations paramétriques ou graphiques générées dans un premier temps et ultérieurement.

10. La méthode selon l'une quelconque des revendications précédentes, où la représentation paramétrique ou graphique est générée à partir du premier signal d'IDT en continu sur une période d'au moins 24 heures consécutive à une intervention chirurgicale.

11. La méthode selon l'une quelconque des revendications précédentes, comprenant également l'apport d'une valeur seuil pour la représentation paramétrique ou graphique générée, et où
o la représentation paramétrique ou graphique générée est surveillée ; et
o un état d'alarme est déclenché si la représentation paramétrique ou graphique générée dépasse la valeur seuil.

12. La méthode selon l'une quelconque des revendications précédentes, où la représentation paramétrique ou graphique est générée sur une période de temps incluant ou suivant l'administration intraveineuse d'un fluide ou d'un médicament influant sur la fonction cardiaque hypokinétique globale, et où la méthode comprend en outre la surveillance d'un changement de la fonction cardiaque hypokinétique globale pendant cette période de temps.

13. Unité de soins postopératoires (20) permettant d'analyser automatiquement un signal ultrasonore d'imagerie Doppler tissulaire (IDT), l'unité comprenant
un moyen (21) de réception d'un premier signal d'IDT obtenu à l'aide d'une sonde Doppler miniaturisée devant être positionné dans une zone d'irrigation artérielle coronaire appropriée sur une partie de la surface externe du myocarde et d'un électrocardiogramme enregistré simultanément ;
un écran d'affichage (27) ; et
une unité de traitement électronique (20) comprenant un moyen logiciel configuré pour :
- extraire à partir du premier signal d'IDT un tracé correspondant à au moins l'un des éléments suivants : vitesse tissulaire, tension tissulaire ou taux de tension tissulaire ;
- corréler le tracé extrait avec l'électrocardiogramme pour
o affecter des parties du tracé extrait à des cycles cardiaques individuels ; et pour
o définir des sous-sections d'un cycle cardiaque dans le tracé extrait correspondant au début de la phase systolique et à la phase post-systolique ;
- lire, dans au moins la phase post-systolique du tracé extrait, une vitesse et/ou une tension et/ou un taux de tension ;
- générer une représentation paramétrique ou graphique qui est fonction de l'une de ces lectures et qui indique une ischémie myocardique régionale ou est corrélée avec une fonction cardiaque hypokinétique globale ; et
- afficher la représentation paramétrique ou graphique générée.

14. L'unité de soins postopératoires selon la revendication 13, comprenant également des moyens d'entrée (28, 40) permettant à un opérateur de définir une valeur seuil pour la représentation paramétrique ou graphique générée et des moyens (40, 41) de générer une alarme ; et où l'unité de traitement électronique comprend également un moyen logiciel pour surveiller la représentation paramétrique ou graphique générée et activer les moyens de générer une alarme si la représentation paramétrique ou graphique générée dépasse la valeur seuil.
